# EUROPÄISCHE PATENTSCHRIFT

(11) **EP 3 102 562 B1**
(45) Veröffentlichungstag und Bekanntmachung des Hinweises auf die Patenterteilung: **31.01.2018**
(21) Anmeldenummer: 15703554.4
(22) Anmeldetag: 03.02.2015
(51) Int. Cl.: C07C 253/34, C07C 253/10, C07C 255/04, C07C 209/48, C07C 211/12

(54) **VERFAHREN ZUR AUFREINIGUNG VON ADIPODINITRIL (ADN)**
METHOD FOR THE PURIFICATION OF ADIPODINITRILE (ADN)
PROCÉDÉ DE NETTOYAGE D'ADIPODINITRILE (ADN)

(30) Priorität: 07.02.2014 EP 14154300
(43) Veröffentlichungstag der Anmeldung: 14.12.2016
(73) Patentinhaber: BASF SE, 67056 Ludwigshafen am Rhein (DE)
(72) Erfinder: LUYKEN, Hermann, 67056 Ludwigshafen (DE); PFAB, Peter, 67056 Ludwigshafen (DE); JUNGKAMP, Tim, 69207 Sandhausen (DE)
(86) Internationale Anmeldenummer: PCT/EP2015/052127
(87) Internationale Veröffentlichungsnummer: WO 2015/117933

(56) Entgegenhaltungen:
- WO-A1-2005/019160
- DE-B- 1 268 611
- DATABASE WPI Week 201367 Thomson Scientific, London, GB; AN 2013-Q93604 XP002738213, & CN 202 942 671 U (ANSHAN GUORUI CHEMICAL) 22. Mai 2013 (2013-05-22)

## Beschreibung

Die vorliegende Erfindung betrifft ein Verfahren zur Aufreinigung von Adipodinitril (ADN), wobei Roh-ADN in eine Rektifikationsvorrichtung (R1) eingespeist wird. Die Rektifikationsvorrichtung (R1) umfasst einen ersten und vorzugswese auch einen zweiten Seitenabzug, wobei sich der erste Seitenabzug unterhalb und der gegebenenfalls vorhandene zweite Seitenabzug oberhalb der Einspeisungsstelle des Roh-ADNs befinden. Über den ersten Seitenabzug wird ein gasförmiger Strom abgezogen, der ADN enthält, während über den gegebenenfalls vorhandenen zweiten Seitenabzug unerwünschte Nebenprodukte wie 1-Amino-2-cyanocyclopenten (ACCP) abgezogen werden, die häufig bei der ADN-Herstellung anfallen und folglich im Roh-ADN enthalten sein können. Der aus dem ersten Seitenabzug von (R1) stammende gasförmige Strom wird in eine zweite Rektifikationsvorrichtung (R2) eingespeist. In (R2) wird ADN von verbliebenen Hochsiedern und gegebenenfalls sonstigen vorhandenen Nebenprodukte abgetrennt, wobei über Kopf aus (D2) reines ADN entnommen wird. Vorzugsweise wird im erfindungsgemäßen Verfahren Roh-ADN eingesetzt, das aus einer Umsetzung von Butadien mit Blausäure (HCN) stammt.

Adipodinitril kann großtechnisch prinzipiell durch 3 unterschiedliche Verfahren hergestellt werden. Im einzelnen erfolgt dies durch i) Umsetzung von Adipinsäure mit Ammoniak, ii) durch Dimerisierung von Acrylnitril oder iii) durch Hydrocyanierung von Butadien mit Blausäure.

In der dritten, bevorzugten Verfahrensalternative wird Adipodinitril aus Butadien und Blausäure in einem mehrstufigen Verfahren hergestellt. Im ersten Verfahrensschritt wird Butadien mit Blausäure in Gegenwart von Nickel(0)-Phosphorligand-Komplexen zu Gemischen umgesetzt, die überwiegend 3-Pentennitril und 2-Methyl-3-butennitril enthalten.3-Pentennitril und 2-Methyl-3-butennitril werden destillativ getrennt. 2-Methyl-3-butennitril wird zu 3-Pentennitril isomerisiert. Im zweiten Verfahrensschritt wird 3-Pentennitril mit Blausäure in Gegenwart von Nickel(0)-Phosphorligandkomplexen und zusätzlich einer Lewissäure zu Adipodinitril hydrocyaniert (siehe auch Hans-Jürgen Arpe, Industrielle Organische Chemie, 6. Auflage (2007), Wiley VCH-Verlag, Seiten 272 bis 273 oder WO 2006/042675). Adipodinitril fällt dabei zunächst als sogenanntes Roh-ADN an.

WO 2005/073167 zeigt, dass zur Herstellung von ADN durch Hydrocyanierung von 1,3-Butadien in einem zweistufigen Verfahren die als Katalysator eingesetzten Nickel(0)-Phosphorligandkomplexe monodentate und bidentate Phosphorliganden aus der Gruppe der Phosphite, Phosphinite und Phosphonite aufweisen.

Dieses Roh-ADN, das auch nach einem anderen Verfahren als über den Butadien-Weg hergestellt werden kann, wird in der Industrie häufig direkt weiterverarbeitet, indem es nach destillativer Reinigung (unter Erhalt von reinem ADN) in Gegenwart eines Hydrierkatalysators, wie z. B. Raney-Nickel, zu Hexamethylendiamin (HMD) hydriert wird. Dessen Umsetzung mit Adipinsäure führt über das sogenannte AH-Salz durch thermische Polykondensation zu Polyamid 6.6.

Roh-Adipodinitril enthält neben dem Hauptprodukt Adipodinitril eine Reihe von Nebenprodukten, wobei die Art und/oder Menge der jeweiligen Nebenprodukte vom gewählten ADN-Herstellungsverfahren abhängen. Als Nebenprodukte fallen insbesondere verzweigte Dinitrile wie 2-Methylglutaronitril (2-MGN), 2-Ethylsuccinonitril (2-ESN) und 1-Amino-2-cyanocyclopenten (ACCP) an. Die verzweigten Dinitrile entstehen beispielsweise bei der Hydrocyanierung von 3-Pentennitril. 1-Amino-2-cyanocyclopenten bildet sich aber auch durch intramolekulare Cyclisierung von Adipodinitril, insbesondere bei höheren Temperaturen. 1-Amino-2-cyanocyclopenten (ACCP, im Englischen auch als "ICCP" abgekürzt) ist ein Tautomer von 1-Imino-2-cyanocyclopentan (CCPI oder CPI) (P. Marion et al., Heterogeneous Catalysis and Fine Chemicals III (1993), Seite 293):

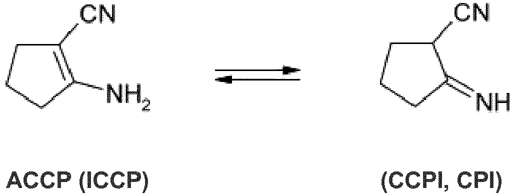

Destillativ nicht abgetrennte Nebenkomponenten (Dinitrile und/oder Amononitrile) werden bei der ADN-Hydrierung zu Diaminen hydriert, die bei der Polykondensation zu Polyamiden zu vorzeitigem Kettenabbruch und dadurch zu Vergilbung der Polyamide führen (siehe auch DE-A 1 268 611, Spalte 1, Zeilen 19 bis 27). Daher sollten die Nebenkomponenten schon vor der ADN-Hydrierung möglichst vollständig abgetrennt werden. Aus beispielsweise DE-A 1 268 611 ist bekannt, im Falle von 1-Amino-2-cyanocyclopenten die gebildete Menge durch Senkung der Sumpftemperaturen bei der ADN-Destillation zu verringern.

DE-A 1 268 611 offenbart ferner ein Verfahren zur Reinigung von Adipodinitril durch Destillation unter Verwendung von drei Kolonnen. ADN wird aus dem Sumpf der zweiten Destillationskolonne abgezogen. Die dritte Kolonne ist erforderlich, um die großen Mengen an ADN zurückzugewinnen, die bei der Destillation in der zweiten Kolonne über Kopf mit den Niedrigsiedern abgezogen werden. Das in DE-A 1 268 611 eingesetzte Roh-ADN stammt aus der Umsetzung von Adipinsäure mit Ammoniak. Aus Process Economics Program Report No. 54 B, Nylon 6.6 Supp.B, September 1987,Seiten 201 bis 214 in Verbindung mit 571 bis 575 (Figure 10.1) ist bekannt, durch Hydrocyanierung hergestelltes Roh-ADN destillativ in drei Kolonnen in Rein-ADN zu überführen. Ausgehend von Roh-Adipodinitril, das über den Butadien-Weg hergestellt wurde, wird in diesem Verfahren in einer ersten Kolonne (C-303, ADN-column) als Kopfprodukt (Strom 42) nicht umgesetztes 3-Pentennitril abgetrennt. Das Sumpfprodukt der ersten Kolonne (Strom 41) wird einer zweiten Kolonne (C-401, Isomer column) zugeführt, in der über Kopf ein Gemisch aus 2-Methylglutaronitril und 2-Ethylsuccinonitril abgetrennt wird. Das Sumpfprodukt der zweiten Kolonne (Strom 53) wird einer dritten Kolonne (C-402, Purification column) zugeführt, in der über Kopf Adipodinitril abgezogen wird. Die Kopftemperatur der dritten Kolonne beträgt 369°F (187 °C) bei 27 mm Hg (36 mbar), die Sumpftemperatur 400°F (204 °C). Das Sumpfprodukt der dritten Kolonne besteht aus Hochsiedern, die ausgeschleust und gegebenenfalls entsorgt werden. Nachteilig an dem beschriebenen Verfahren ist, dass für die destillative Herstellung von reinem Adipodinitril drei Destillationskolonnen benötigt werden. Weiterhin ist nachteilig, dass die Sumpftemperatur der dritten Kolonne mehr als 200°C beträgt und dadurch deutliche Mengen an Adipodinitril in 1-Amino-2-cyanocyclopenten umgewandelt und nach Hydrierung zu dem von Hexamethylendiamin schwer abtrennbaren 2-Aminomethylcyclopentylamin umgesetzt werden.

In WO 2005/019160 A1 wird offenbart, dass Roh-Adipodinitril (Strom 13), hergestellt durch Hydrocyanierung von Butadien und 3-Pentennitril mit Blausäure, einer Kolonne 14 zugeführt wird. Als Kopfprodukt der Kolonne (Strom 45) werden ganz überwiegend Pentennitrile, als Sumpfprodukt (Strom 17) Hochsieder entnommen. Aus einem Seitenabzug der Kolonne 14 (Strom 19) werden Dinitril-Gemische, umfassend Adipodinitril, 2-Methylglutaronitril und 2-Ethylsuccinonitril, entnommen. Um aus diesem Dinitril-Gemisch wiederum ADN in reiner Form isolieren zu können, sind weitere aufwändige Trennungsschritte erforderlich, deren Durchführung in WO 2005/019160 A1 jedoch nicht konkret offenbart sind.

Nachteilig an dem in WO 2005/019160 A1 beschriebenen Verfahren ist insbesondere, dass aus Adipodinitril (Kp=303 °C/1013 mbar) gebildetes 1-Amino-2-cyanocyclopenten mit 285 °C/1013 mbar einen Siedepunkt zwischen ADN und seinen verzweigten Isomeren 2-MGN (Kp=274 °C/1013 mbar) und 2-ESN (Kp=265 °C/1013 mbar) besitzt. Aus Seitenabzug 19 wird im Verfahren gemäß WO 2005/019160 A1 demnach ein Gemisch entnommen, das außer ADN auch noch erhebliche Mengen an 2-MGN, 2-ESN und ACCP enthält. Aus diesem Gemisch muss jedoch das Zielprodukt Adipodinitril in reiner Form abgetrennt werden. Hierzu sind somit weitere Destillationskolonnen erforderlich.

Die der vorliegenden Erfindung zugrunde liegende Aufgabe besteht in der Bereitstellung eines neuen Verfahrens zur Aufreinigung von Adipodinitril (ADN) aus Roh-ADN.

Gelöst wird die Aufgabe erfindungsgemäß durch ein Verfahren zur Aufreinigung von Adipodinitril (ADN) umfassend die folgenden Schritte a) bis c):
a) Einspeisung eines Stromes (S1) enthaltend Roh-Adipodinitril (Roh-ADN) in eine Rektifikationsvorrichtung (R1),
b) Abtrennen eines gasförmigen Stromes (S2) über einen ersten Seitenabzug von (R1), wobei sich der erste Seitenabzug von (R1) unterhalb der Einspeisungsstelle des Stromes (S1) nach (R1) befindet und wobei der gasförmige Strom (S2) ADN enthält, und
c) Einspeisung des gasförmigen Stroms (S2) in eine zweite Rektifikationsvorrichtung (R2), wobei aus (R2) über Kopf ein an Hochsiedern (HS) abgereicherter ADN-Strom (S7) und über Sumpf ein an Hochsiedern (HS) angereicherter flüssiger Strom (S6) abgezogen wird.

Durch das erfindungsgemäße Verfahren kann in vorteilhafter Weise ADN, insbesondere ADN mit hoher Reinheit, hergestellt werden. Im erfindungsgemäßen Verfahren kann jedes beliebige Roh-ADN aufgereinigt werden, unabhängig von dessen konkretem Herstellungsverfahren. Durch das erfindungsgemäße Verfahren werden insbesondere die vorstehend im Zusammenhang mit der Aufreinigung von Roh-ADN, das über den Butadien-Weg hergestellt wurde, beschriebenen Nachteile ausgeräumt.

Ein weiterer Vorteil des erfindungsgemäßen Verfahrens ist, dass es sehr flexibel gehandhabt werden kann. Abhängig von dem eingesetzten Roh-ADN kann das erfindungsgemäße Verfahren weiter optimiert werden. Ist beispielsweise im eingesetzte Roh-ADN ACCP enthalten, wird das erfindungsgemäße Verfahren in vorteilhafter Weise so durchgeführt, dass die Rektifikationsvorrichtung (R1) über einen zweiten Seitenabzug verfügt. Über diesen zweiten Seitenabzug von (R1), der sich oberhalb der Einspeisungsstelle des Stromes (S1) nach (R1) befindet, kann ein Strom (S3) aus (R1) abgetrennt werden, wobei der Strom (S3) 1-Amino-2-cyanocyclopenten (ACCP) sowie gegebenenfalls vorhandene weitere unerwünschte Nebenprodukte wie insbesondere 2-MGN und/oder 2-ESN enthält.

Als weiterer Vorteil des erfindungsgemäßen Verfahrens kann auch angesehen werden, dass im Roh-ADN in der Regel vorhandene Hochsieder, vorzugsweise flüchtige Hochsieder, auf einfache Weise vom ADN abgetrennt werden können. Diese vorzugsweise flüchtigen Hochsieder werden zunächst über den gasförmigen Seitenabzug (aus dem Abtriebsteil, also unterhalb der Einspeisungsstelle des Stroms (S1)) aus der Rektifikationsvorrichtung (R1) gemeinsam mit (der Hauptmenge) des ADN abgezogen. Diese flüchtigen Hochsieder können in nur einer einzigen weiteren Kolonne (Rektifikationsvorrichtung (R2)) effektiv abgereichert/abgetrennt werden, so dass aus dem Kopf von (R2) reines ADN erhalten werden kann. Die Rektifikationsvorrichtung (R2) kann ohne einen zusätzlichen Verdampfer betrieben werden, was ebenfalls einen Vorteil des erfindungsgemäßen Verfahrens darstellt.

Ein genereller Vorteil des erfindungsgemäßen Verfahrens ist jedoch, dass Apparaturen, insbesondere Rektifikations- und/oder Destillationskolonnen, eingespart werden. Generell anfallende störende Nebenprodukte, insbesondere ACCP, 2-MGN und/oder 2-ESN, können im erfindungsgemäßen Verfahren überwiegend in einer Fraktion abgetrennt und gegebenenfalls gemeinsam entsorgt werden. Dabei kann auch das in DE-A 1 268 611 angesprochene Problem der Feststoffbildung vermieden werden, weil beispielsweise das bei Raumtemperatur feste ACCP mit den anderen Nebenprodukten (in der Regel in flüssiger Form) als Bestandteil des Stromes (S3) aus dem Verfahren ausgeschleust wird, so dass keine Verstopfungen des Seitenabzuges auftreten. Durch geeignete Parametereinstellung, insbesondere von Druck- und Temperatur, kann das erfindungsgemäße Verfahren so gesteuert werden, dass unabhängig von den weiteren Komponenten des Stromes (S3) keine Verstopfungen durch etwaig ausfallendes ACCP auftreten. Vorzugsweise wird das erfindungsgemäße Verfahren aber so durchgeführt, dass im Strom (S3), dem zweiten Seitenabzug aus der Rektifikationsvorrichtung (R1), außer ACCP noch weitere Nebenprodukte, insbesondere verzweigte Dinitrile wie 2-ESN und/oder 2-MGN, enthalten sind, da sich ACCP in den verzweigten Dinitrilen gut löst.

Ein weiterer Vorteil des erfindungsgemäßen Verfahrens ist darin zu sehen, dass bei der destillativen Aufreinigung von Roh-ADN reines ADN, also ADN mit einem Reinheitsgrad von mindestens 99,0 %, insbesondere ADN mit hoher Reinheit, erhalten wird, wofür prinzipiell nur zwei Rektifikationsvorrichtungen (D1 und D2) erforderlich sind. Unter ADN mit hoher Reinheit versteht man einen Strom mit einem Gehalt an ADN von größer als 99.0 %, insbesondere von größer als 99.5 %. Außerdem wird in der zweiten Rektifikationsvorrichtung (R2) durch Entnahme von gasförmigem ADN aus Rektifikationseinrichtung (R1) ein Verdampfer eingespart.

Weiterhin ist es vorteilhaft, dass im erfindungsgemäßen Verfahren aufgrund entsprechender Rückführungen kein oder nur sehr wenig ADN verloren geht. Das im Roh-ADN enthaltene ADN kann nahezu vollständig zu reinem ADN aufgereinigt werden.

Nachfolgend wird das erfindungsgemäße Verfahren zur Aufreinigung von ADN näher definiert.

In Schritt a) des erfindungsgemäßen Verfahrens erfolgt die Einspeisung eines Stromes (S1) enthaltend Roh-Adipodinitril (Roh-ADN) in eine Rektifikationsvorrichtung (R1). Der Strom (S1) kann dampfförmig oder flüssig sein.

Roh-ADN als solches ist dem Fachmann bekannt und enthält ADN (als solches). Wie vorstehend bereits ausgeführt, können in Abhängigkeit von konkreten Herstellungsverfahren des Roh-ADN zahlreiche weitere Komponenten, wie Nebenprodukte, Edukte oder sonstige Verbindungen, im Roh-ADN enthalten sein. Der ADN-Anteil im Strom (S1) des erfindungsgemäßen Verfahrens sowie die Anteile aller sonstigen Komponenten können beliebig sein. Vorzugsweise ist der ADN-Anteil in Strom (S1) höher als 30 Gew.-%, besonders bevorzugt höher als 40 Gew.-%, ganz besonders bevorzugt höher als 50 Gew.-%.

Beispiele für weitere Komponenten (neben ADN als solchem), die in Roh-ADN gemäß Strom (S1) und gegebenenfalls in sonstigen Strömen des erfindungsgemäßen Verfahrens wie beispielsweise dem weiter unten definierten Strom (S8) enthalten sein können, sind nachfolgend beispielhaft aufgeführt:
Bei der Hydrocyanierung von 3-Pentennitril mit Blausäure zu Adipodinitril in Gegenwart von Nickel(0)-Phosphorligand-Komplexen und Lewis-Säuren wie z. B. Zinkchlorid entstehen komplexe Produktgemische. Sie enthalten das Zielprodukt Adipodinitril, daneben die verzweigten Dinitrile 2-Methylglutaronitril (2-MGN) und 2-Ethylsuccinonitril (2-ESN). Weiterhin nicht umgesetzte oder durch Isomerisierung entstandene Pentennitrile wie trans-3-Pentennitril, cis-3-Pentennitril, 4-Pentennitril,cis-2-Pentennitril, trans-2-Pentennitril (Siedebereich der Pentennitrile 127 bis 146 °C/1013 mbar), aus Adipodinitril entstandenes 1-Amino-2-cyanocyclopenten, Nickel(0)-Phorphorligand-Komplexe, freie Liganden der Nickel(0)-Komplexe und Hochsieder (HS).

Unter Hochsiedern sind im Rahmen der vorliegenden Erfindung Verbindungen zu verstehen, die einen höheren Siedepunkt als Adipodinitril besitzen. Diese können je nach Art einen messbaren Dampfdruck (z.B. Dimere) oder keinen Dampfdruck (z. B. Salze) haben.

Die Hochsieder können wiederum in flüchtige Hochsieder sowie nicht-flüchtige Hochsieder unterschieden werden. Wie aus den nachfolgenden Ausführungen ersichtlich, werden die nicht-flüchtigen Hochsieder (die Hauptmenge oder vorzugsweise vollständig) vorzugsweise bereits aus dem Roh-ADN abgetrennt, bevor dieses in die Rektifikationsvorrichtung (R1) eingespeist wird. Flüchtige Hochsieder werden hingegen vorzugsweise (vollständig oder zumindest größtenteils) unter Einsatz der Rektifikationsvorrichtung (R2) von ADN abgetrennt. Diese (flüchtigen) Hochsieder sind also gemeinsam mit ADN im gasförmigen Strom (S2) enthalten, der über den ersten Seitenabzug von (R1) aus dieser abgetrennt wird.

Flüchtige Hochsieder können z. B. monodentate oder bidentate Phosphorliganden sowie dimere Pentennitrile oder Dinitrile sein, nichtflüchtige Hochsieder können z. B. Zinkchlorid oder Eisenchlorid sein.

Die Nickel(0)-Phosphorligand-Komplexe und die entsprechenden freien Phosphorligand-Komplexe werden vorzugsweise durch Extraktion mit Kohlenwasserstoffen aus den Produktgemischen abgetrennt (wie beispielhaft in EP 1 817 108 B1 ausgeführt).

In Schritt b) des erfindungsgemäßen Verfahrens erfolgt das Abtrennen eines gasförmigen Stromes (S2) über einen ersten Seitenabzug von (R1), wobei sich der erste Seitenabzug von (R1) unterhalb der Einspeisungsstelle des Stromes (S1) nach (R1) befindet und wobei der gasförmige Strom (S2) ADN enthält. Vorzugsweise wird der gasförmige Strom (S2) über den ersten Seitenabzug aus dem Sumpf von (R1) abgezogen.

Der Anteil an ADN im gasförmigen Strom (S2) ist normalerweise höher als 80 Gew.-%, besonders bevorzugt höher als 90 Gew.-%, ganz besonders bevorzugt höher als 95 Gew.-%. Wie nachfolgend ausgeführt, können im gasförmigen Strom (S2) neben ADN auch weitere Komponenten, wie Hochsieder, insbesondere flüchtige Hochsieder, enthalten sein.

Schritt c) des erfindungsgemäßen Verfahrens erfordert die Einspeisung des gasförmigen Stroms (S2) in eine zweite Rektifikationsvorrichtung (R2), wobei aus (R2) über Kopf ein an Hochsiedern (HS) abgereicherter ADN-Strom (S7) und über Sumpf ein an Hochsiedern (HS) angereicherter flüssiger Strom (S6) abgezogen wird.

In anderen Worten ausgedrückt bedeutet dies, dass durch Einsatz der zweiten Rektifikationsvorrichtung (R2) das Zielprodukt ADN in aufgereinigter Form aus dem erfindungsgemäßen Verfahren gewonnen werden kann. Sofern im Sumpf von (R2) noch ADN enthalten ist, kann dies beispielsweise in die Rektifikationsvorrichtung (R1) rückgeleitet werden, wie nachfolgend weiter ausgeführt.

Die Sumpftemperatur der Rektifikationsvorrichtung (R1) beträgt normalerweise 120 bis 240 °C, bevorzugt 150 bis 220 °C, besonders bevorzugt 160 bis 190 °C. Der Kopfdruck von (R1) beträgt normalerweise 3 bis 250, bevorzugt 10 bis 150, besonders bevorzugt 15 bis 50 mbar.

Die Sumpftemperatur der Rektifikationsvorrichtung (R2) beträgt normalerweise 120 bis 240 °C, bevorzugt 150 bis 220 °C, besonders bevorzugt 160 bis 190 °C.

Der Kopfdruck der Rektifikationsvorrichtung (R2) beträgt normalerweise 3 bis 250 mbar, bevorzugt 10 bis 150 mbar, besonders bevorzugt 15 bis 50 mbar.

Die erfindungsgemäßen Rektifikationsvorrichtungenen (R1) und/oder (R2) können in jeder geeigneten, dem Fachmann bekannten Form durchgeführt werden. Für diese Rektifikationsvorrichtungen sind Apparaturen geeignet, wie sie beispielsweise in: Kirk-Othmer, Encyclopedia of Chemical Technology, 4. Auflage, Band 8, John Wiley and Sons, New York 1996, Seiten 334 bis 348 beschrieben sind, wie Siebbodenkolonnen, Glockenbodenkolonnen, Packungskolonnen und Füllkörperkolonnen.

Bevorzugt weisen die Rektifikationsvorrichtungen (R1) und/oder (R2) Einbauten zur Erhöhung der Trennleistung auf. Die Einbauten können beispielsweise als geordnete Packung vorliegen, beispielsweise als Blechpackung wie Mellapak 250 Y oder Montz Pak,Typ B1-250.Es kann auch eine Packung mit geringerer oder erhöhter spezifischer Oberfläche vorliegen oder es kann eine Gewebepackung oder eine Packung mit anderer Geometrie als Mellapak 252 Y verwendet werden. Vorteilhaft bei der Verwendung dieser Einbauten ist der geringe Druckverlust und der geringe spezifische Flüssig-Hold-up im Vergleich mit beispielsweise Ventilböden. Die Einbauten können in ein oder mehreren Betten vorliegen.

Die Zahl der theoretischen Böden in der Rektifikationsvorrichtung (R1) beträgt normalerweise 15 bis 50, bevorzugt 20 bis 40, besonders bevorzugt 25 bis 35 und/oder in der Rektifikationsvorrichtung (R2) normalerweise 3 bis 20, bevorzugt 5 bis 17, besonders bevorzugt 8 bis 12.

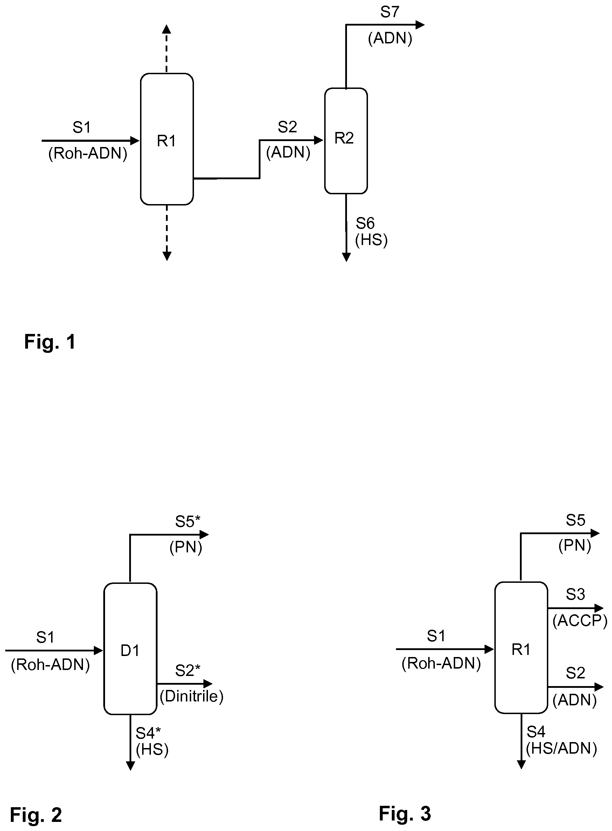

Das erfindungsgemäße Verfahren umfassend die Schritte a) bis c) ist in seiner Basisversion in der Figur 1 verdeutlicht. Zu den in Figur 1 aufgeführten Strömen (S1), (S2), (S6) und (S7) sind zur Verdeutlichung in Klammern diejenigen Komponenten aufgeführt, die für das erfindungsgemäße Verfahren/die Auftrennung wesentlich sind. Wie vorstehend oder nachfolgend detailliert aufgeführt, können in den jeweiligen Strömen auch noch weitere Komponenten enthalten sein. Die beiden am Kopf bzw. am Sumpf von (R1) aufgeführten gestrichelten Pfeile symbolisieren zusätzliche Ströme, die vorzugsweise im Rahmen der vorliegenden Erfindung ebenfalls aus der Rektifikationsvorrichtung (R1) abgezogen werden. Diese Ströme werden im nachfolgenden Text im Zusammenhang mit den Verfahrensschritten e) und f) sowie beispielsweise der Figur 3 weiter verdeutlicht.

Im Rahmen des erfindungsgemäßen Verfahrens ist es weiterhin bevorzugt, dass das Roh-Adipodinitril (Roh-ADN) in Strom (S1) Adipodinitril (ADN), Pentennitrile (PNs), Hochsieder (HS), 1-Amino-2-cyanocyclopenten (ACCP), 2-Methylglutarnitril (2-MGN) und 2-Ethylsuccinonitril (2-ESN) enthält.

Weiterhin ist es im Rahmen des erfindungsgemäßen Verfahrens bevorzugt, dass der gasförmige Strom (S2) in den Sumpf von (R2) eingespeist wird und aus dem Kopf von (R2) reines ADN abgezogen wird.

Weiterhin ist es erfindungsgemäß bevorzugt, dass die Reinheit des aus dem Kopf von (R2) abgezogenen ADN mindestens 99,0 %, bevorzugt mindestens 99,5 %, besonders bevorzugt mindestens 99,8 % beträgt und/oder die Gesamtmenge an PNs, ACCP, 2-MGN und 2-ESN im aus dem Kopf von (D2) abgezogenen ADN nicht größer als 500 ppm, mehr bevorzugt nicht größer als 200 ppm, insbesondere nicht größer als 100 ppm ist.

Weiterhin ist es im erfindungsgemäßen Verfahren bevorzugt, dass aus dem Sumpf von (R2) der Strom (S6), der Hochsieder (HS) und ADN enthält, abgezogen und ganz oder teilweise in den Sumpf der Rektifikationsvorrichtung (R1) rückgeleitet wird.

Weiterhin ist es erfindungsgemäß bevorzugt, dass die Rektifikationsvorrichtungen (R1) und (R2) unabhängig voneinander Packungskolonnen sind. Ebenfalls ist es bevorzugt, dass die Rektifikationsvorrichtung (R2) keinen Verdampfer hat.

In einer bevorzugten Ausführungsform der vorliegenden Erfindung wird außer den vorstehend beschriebenen Schritten a) bis c) mindestens einer der nachfolgend beschriebenen Schritte d), e) oder f) durchgeführt. Prinzipiell kann das erfindungsgemäße Verfahren gemäß den Schritten a) bis c) mit jedem einzelnen Schritt d), e) und/oder f) kombiniert werden. Besonders bevorzugt wird das erfindungsgemäße Verfahren jedoch als Kombination der Schritte a) bis f) durchgeführt. Die einzelnen Schritte d), e) und f) sind wie folgt definiert:

Gemäß Schritt d) erfolgt das Abtrennen eines Stromes (S3) über einen zweiten Seitenabzug der Rektifikationsvorrichtung (R1), wobei sich der zweite Seitenabzug von (R1) oberhalb der Einspeisungsstelle des Stromes (S1) nach (R1) befindet und wobei der Strom (S3) 1-Amino-2-cyanocyclopenten (ACCP) enthält.

Gemäß Schritt e) erfolgt das Abtrennen eines Stromes (S5) über den Kopf der Rektifikationsvorrichtung (R1), wobei der Strom (S5) Pentennitrile (PNs), insbesondere 3-Pentennitril (3-PN) enthält.

Gemäß Schritt f) erfolgt das Abtrennen eines Stromes (S4) aus dem Sumpf der Rektifikationsvorrichtung (R1), wobei der Strom (S4) Hochsieder (HS) enthält.

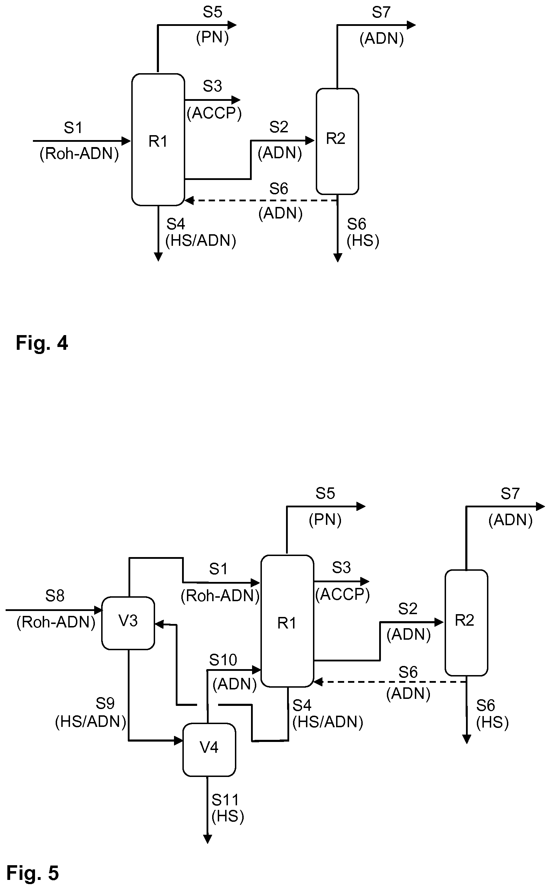

Besonders bevorzugt ist im Rahmen dieser Ausführungsform der vorliegenden Erfindung dass
i) der Strom (S4) Hochsieder und ADN enthält und flüssig ist, und/oder
ii) der Strom (S3) ACCP, 2-MGN und 2-ESN enthält und flüssig ist, und/oder
iii) der gasförmige Strom (S2) ADN, Hochsieder und nicht mehr als 500 ppm an PNs, ACCP, 2-MGN und/oder 2-ESN enthält, vorzugsweise ist die Gesamtmenge an PNs, ACCP, 2-MGN und 2-ESN in (S2) nicht größer als 500 ppm, mehr bevorzugt nicht größer als 200 ppm, insbesondere nicht größer als 100 ppm. Die vorstehend beschriebenen bevorzugten Ausgestaltungen/Ausführungsformen der vorliegenden Erfindung werden in den Figuren 3 und 4 verdeutlicht. In diesen sowie in allen sonstigen Figuren der vorliegenden Erfindung haben - soweit nicht anders ausgeführt - die Abkürzungen, Pfeile sowie sonstigen Symbole eine sinngemäße Bedeutung wie vorstehend für Figur 1 ausgeführt. Figur 3 verdeutlicht eine bevorzugte Ausgestaltung der Rektifikationsvorrichtung (R1), in der die drei optionalen Schritte d), e) und f) mitberücksichtigt sind. Figur 4 zeigt wiederum, wie die bevorzugte Ausführungsform der Rektifikationsvorrichtung (R1) in das Gesamtverfahren integriert ist. Der gestrichelte Pfeil soll darstellen, dass optional auch eine Rückführung von gegebenenfalls im Strom (S6) enthaltenem ADN in die Rektifikationsvorrichtung (R1) denkbar ist. Sofern keine Rückleitung des Stromes (S6) aus (R2) in (R1) durchgeführt wird, werden die über den Strom (S6) abgetrennten Hochsieder, vorzugsweise flüchtigen Hochsieder, vorzugsweise vollständig aus dem erfindungsgemäßen Verfahren abgetrennt. Beispielsweise können die Komponenten des Stromes (S6) entsorgt werden und/oder einer sonstigen Verwendung zugeführt werden. Sofern in Strom (S6) ADN in nennenswertem Umfang enthalten ist, wird erfindungsgemäß vorzugsweise eine zumindest teilweise, vorzugsweise vollständige Rückführung des Stromes (S6) aus (R2) nach (R1) durchgeführt.

Zur Verdeutlichung der Vorteile des erfindungsgemäßen Verfahrens, insbesondere im Zusammenhang mit den Figuren 3 und 4, ist zum Vergleich in Figur 2 eine sinngemäße Destillationsanordnung aus dem Stand der Technik gemäß WO 2005/019160 aufgeführt. Bei diesem Verfahren des Standes der Technik werden aus einem Seitenabzug der entsprechenden Destillationsvorrichtung (D1) sämtliche Dinitrile in flüssiger Form entnommen. Die Abtrennung des gasförmigen Stromes enthaltend ADN ist im Verfahren gemäß WO 2005/019160 jedoch nicht vorgesehen.

Weiterhin ist es im Rahmen der vorliegenden Erfindung bevorzugt, dass das im Strom (S1) enthaltene Roh-ADN bereits vor Eintritt in die Rektifikationsvorrichtung (R1) einer zusätzlichen Aufreinigung unterzogen wird, insbesondere werden dabei Hochsieder, vorzugsweise nicht-flüchtige Hochsieder, aus dem eingesetzten Roh-ADN abgetrennt. In diesem Zusammenhang können auch Ströme wie der hochsieder- sowie der ADN-haltige Strom (S4) aus dem Verfahren (einem späteren Verfahrensschritt) in einem solchen ersten Abtrennungsschritt rückgeleitet werden.

Vorzugsweise wird in diesem Zusammenhang ein Strom (S8), der Roh-ADN und Hochsieder (HS) enthält, in eine Verdampfungsvorrichtung (V3) eingeleitet wird, wobei aus (V3) ein Strom (S1'), der gegenüber dem Strom (S8) an nicht-flüchtigen Hochsiedern (HS) abgereichert ist, ausgeleitet und in die Rektifikationsvorrichtung (R1) weitergeleitet wird, und wobei aus (V3) ein Strom (S9) ausgeleitet wird, der nicht-flüchtige Hochsieder enthält und gegenüber dem Strom (S8) an ADN abgereichert ist.

Als Verdampfungsvorrichtung (V3) kommen beispielsweise einstufige Verdampfer wie Fallfilmverdampfer, Dünnschichtverdampfer, Flashverdampfer, Mehrphasenwendelrohrverdampfer, Naturumlaufverdampfer oder Zwangsumlaufentspannungsverdampfer in Frage.

Weiterhin ist es bevorzugt, dass der Strom (S9) aus dem Sumpf der Verdampfungsvorrichtung (V3) ausgeleitet und in eine Verdampfungsvorrichtung (V4) weitergeleitet wird, wobei aus (V4) ein Strom (S10), der gegenüber dem Strom (S9) an nicht-flüchtigen Hochsiedern abgereichert ist, ausgeleitet und in die Rektifikationsvorrichtung (R1) weitergeleitet und/oder in die Verdampfungsvorrichtung (V3) rückgeleitet wird, und wobei aus (V4) ein Strom (S11), der gegenüber dem Strom (S9) an ADN abgereichert ist, ausgeleitet wird.

Ebenfalls ist es bevorzugt, dass der Strom (S4) aus der Rektifikationsvorrichtung (R1) ganz oder teilweise in die Verdampfungsvorrichtung (V3) und/oder in die Verdampfungsvorrichtung (V4) geleitet wird.

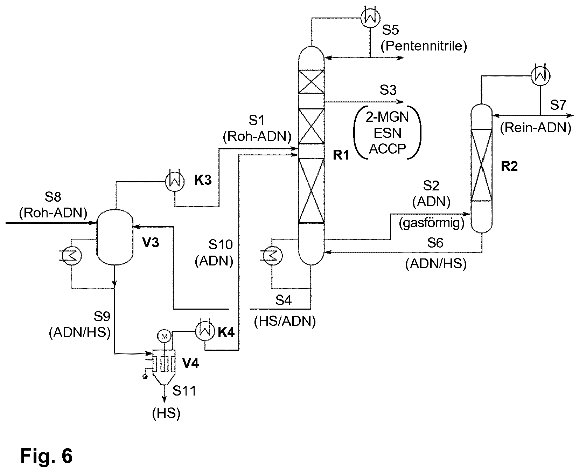

Die vorstehend beschriebenen Ausführungsformen der vorliegenden Erfindung hinsichtlich einer Vorabtrennung von Hochsiedern, vorzugsweise nicht-flüchtigen Hochsiedern, aus dem Roh-ADN vor Einspeisung in die Rektifikationsvorrichtung (R1) ist in den Figuren 5 sowie 6 verdeutlicht. Während in Figur 5 das Verfahren vereinfacht dargestellt ist, sind in Figur 6 weitere bevorzugte Ausgestaltungen wie Kondensatoren, Vorrichtungseinbauten oder Rückleitungen in die Rektifikationsvorrichtungen (R1) und (R2) dargestellt.

In einer weiteren, in Figur 6 dargestellten bevorzugten Verfahrensvariante wird ein Roh-ADN-Strom (S8) in eine Destillations-, Rektifikations- oder vorzugsweise Verdampfungseinrichtung (V3) eingeleitet und verdampft, bevor er als Strom (S1') in Rektifikationsvorrichtung (R1) eingeleitet wird. Hierbei ist es möglich, Strom (S1') gasförmig oder flüssig in (R1) einzuleiten. Bei Verzicht auf einen Kondensator (K3) kann sich der Wärmebedarf des Verdampfers von (R1) verringern. Bei Anwendung eines Kondensators (K3) ist es möglich, einen niedrigeren Druck als in Rektifikationsvorrichtung (R1) in Verdampfungsvorrichtung (V3) einzustellen. Hierdurch ist es möglich, den ADN-Gehalt im Sumpfaustrag von (V3) durch Reduktion des Druckes zu reduzieren.

In einer weiteren, in Figur 6 dargestellten bevorzugten Verfahrensvariante wird der Sumpfaustrag aus Verdampfungsvorrichtung (V3) in einer weiteren Verdampfungsvorrichtung (V4) weiter eingeengt, um ADN über Kopf zu trennen, während Hochsieder in den Sumpf angereichert werden. Als Verdampfungsvorrichtung (V4) können dem Fachmann bekannte Einrichtungen wie Destillationsblasen, Naturumlaufverdampfer, Zwangsumlaufverdampfer oder bevorzugt Fallfilmverdampfer, besonders bevorzugt Dünnschichtverdampfer eingesetzt werden. Vorzugsweise ist der Druck in (V4) niedriger als in (V3). Der Kopfaustrag aus (V4) kann in einem Kondensator (K4) kondensiert und zu Verdampfungsvorrichtung (V3) zurückgeführt oder in Rektifikationsvorrichtung (R1) geleitet werden. Durch den Einsatz von Verdampfungsvorrichtungen (V3) und/oder (V4) kann die Ausbeute an ADN erhöht und dessen Verluste bei der Ausschleusung von Hochsiedern verringert werden.

Weiterhin ist es im erfindungsgemäßen Verfahren bevorzugt, dass das im Roh-Adipodinitril (Roh-ADN) enthaltene ADN durch eine Umsetzung von Butadien mit Blausäure (HCN) hergestellt wird, wobei bei der ADN-Herstellung vorzugsweise in einem ersten Verfahrensschritt Butadien mit HCN in Gegenwart eines Nickel(0)-Phosphorligand-Komplexkatalysators (Ni-P-Kat) zu 3-Pentennitril (3-PN) umgesetzt wird, worauf in einem zweiten Verfahrensschritt 3-PN mit HCN in Gegenwart desselben Ni-P-Kat und einer Lewis-Säure zu ADN umgesetzt wird.

Alternativ kann man auch, wie in WO 2005/073167 beschrieben, für die erste und zweite Hydrocyanierung und die Isomerisierung von 2-Methyl-3-butennitril zu 3-Pentennitrilen den gleichen Nickel(0)-Phosphorligand-Komplex verwenden. In einer bevorzugten Ausführungsform wird jedoch für die erste Hydrocyanierung und die 2-Methyl-3-butennitril-Isomerisierung der gleiche Nickel(0)-Komplex auf Basis eines monodentaten Phosphorliganden, für die zweite Hydrocyanierung ein Nickel(0)-Phosohorligand-Komplex auf Basis eines bidentaten Phosphorliganden verwendet.

Weiterhin ist es im erfindungsgemäßen Verfahren bevorzugt, dass das aufgereinigte ADN in Gegenwart eines Hydrierkatalysators, vorzugsweise in Gegenwart eines Raney-Nickel-Katalysators, zu Hexamethylendiamin (HMD) hydriert wird.

## Patentansprüche

1. Verfahren zur Aufreinigung von Adipodinitril (ADN) umfassend die folgenden Schritte a) bis c)
a) Einspeisung eines Stromes (S1) enthaltend Roh- Adipodinitril (Roh-ADN) in eine Rektifikationsvorrichtung (R1),
b) Abtrennen eines gasförmigen Stromes (S2) über einen ersten Seitenabzug von (R1), wobei sich der erste Seitenabzug von (R1) unterhalb der Einspeisungsstelle des Stromes (S1) nach (R1) befindet und wobei der gasförmige Strom (S2) ADN enthält, und
c) Einspeisung des gasförmigen Stroms (S2) in eine zweite Rektifikationsvorrichtung (R2), wobei aus (R2) über Kopf ein an Hochsiedern (HS) abgereicherter ADN-Strom (S7) und über Sumpf ein an Hochsiedern (HS) angereicherter flüssiger Strom (S6) abgezogen wird.

2. Verfahren gemäß Anspruch 1, umfassend zusätzlich mindestens einen der Schritte d), e) oder f):
d) Abtrennen eines Stromes (S3) über einen zweiten Seitenabzug der Rektifikationsvorrichtung (R1), wobei sich der zweite Seitenabzug von (R1) oberhalb der Einspeisungsstelle des Stromes (S1) nach (R1) befindet und wobei der Strom (S3) 1-Amino-2-cyanocyclopenten (ACCP) enthält und/oder
e) Abtrennen eines Stromes (S5) über den Kopf der Rektifikationsvorrichtung (R1), wobei der Strom (S5) Pentennitrile (PNs), insbesondere 3-Pentennitril (3-PN), enthält und/oder
f) Abtrennen eines Stromes (S4) aus dem Sumpf der Rektifikationsvorrichtung (R1), wobei der Strom (S4) Hochsieder (HS) enthält.

3. Verfahren gemäß Anspruch 1 oder 2, **dadurch gekennzeichnet, dass** das Roh-Adipodinitril (Roh-ADN) in Strom (S1) Adipodinitril (ADN), Pentennitrile (PNs), Hochsieder (HS), 1-Amino-2-cyanocyclopenten (ACCP), 2-Methylglutarnitril (2-MGN) und 2-Ethylsuccinonitril (2-ESN) enthält.

4. Verfahren gemäß einem der Ansprüche 1 bis 3, **dadurch gekennzeichnet, dass**
i) der Strom (S4) Hochsieder und ADN enthält und flüssig ist, und/oder
ii) der Strom (S3) ACCP, 2-MGN und 2-ESN enthält und flüssig ist, und/oder
iii) der gasförmige Strom (S2) ADN, Hochsieder und nicht mehr als 500 ppm an PNs, ACCP, 2-MGN und/oder 2-ESN enthält, vorzugsweise ist die Gesamtmenge an PNs, ACCP, 2-MGN und 2-ESN in (S2) nicht größer als 500 ppm, mehr bevorzugt nicht größer als 200 ppm, insbesondere nicht größer als 100 ppm.

5. Verfahren nach einem der Ansprüche 1 bis 4, **dadurch gekennzeichnet, dass** ein Strom (S8), der Roh-ADN und Hochsieder (HS) enthält, in eine Verdampfungsvorrichtung (V3) eingeleitet wird, wobei aus (V3) ein Strom (S1), der gegenüber dem Strom (S8) an nicht-flüchtigen Hochsiedern (HS) abgereichert ist, ausgeleitet und in die Rektifikationsvorrichtung (R1) weitergeleitet wird, und wobei aus (V3) ein Strom (S9) ausgeleitet wird, der nicht-flüchtige Hochsieder enthält und gegenüber dem Strom (S8) an ADN abgereichert ist.

6. Verfahren nach Anspruch 5, **dadurch gekennzeichnet, dass** der Strom (S9) aus dem Sumpf der Verdampfungsvorrichtung (V3) ausgeleitet und in eine Verdampfungsvorrichtung (V4) weitergeleitet wird, wobei aus (V4) ein Strom (S10), der gegenüber dem Strom (S9) an nicht-flüchtigen Hochsiedern abgereichert ist, ausgeleitet und in die Rektifikationsvorrichtung (R1) weitergeleitet und/oder in die Verdampfungsvorrichtung (V3) rückgeleitet wird, und wobei aus (V4) ein Strom (S11), der gegenüber dem Strom (S9) an ADN abgereichert ist, ausgeleitet wird.

7. Verfahren gemäß einem der Ansprüche 1 bis 6, **dadurch gekennzeichnet, dass** der Strom (S4) aus der Rektifikationsvorrichtung (R1) ganz oder teilweise in die Verdampfungsvorrichtung (V3) und/oder in die Verdampfungsvorrichtung (V4) geleitet wird.

8. Verfahren gemäß einem der Ansprüche 1 bis 7, **dadurch gekennzeichnet, dass** der gasförmige Strom (S2) in den Sumpf von (R2) eingespeist wird und aus dem Kopf von (R2) reines ADN abgezogen wird.

9. Verfahren gemäß einem der Ansprüche 1 bis 8, **dadurch gekennzeichnet, dass** die Reinheit des aus dem Kopf von (R2) abgezogenen ADN mindestens 99,0 %, bevorzugt mindestens 99,5 %, besonders bevorzugt mindestens 99,8 % beträgt und/oder die Gesamtmenge an PNs, ACCP, 2-MGN und 2-ESN im aus dem Kopf von (R2) abgezogenen ADN nicht größer als 500 ppm, mehr bevorzugt nicht größer als 200 ppm, insbesondere nicht größer als 100 ppm ist.

10. Verfahren gemäß einem der Ansprüche 1 bis 9, **dadurch gekennzeichnet, dass** aus dem Sumpf von (R2) der Strom (S6), der Hochsieder (HS) und ADN enthält, abgezogen und ganz oder teilweise in den Sumpf der Rektifikationsvorrichtung (R1) rückgeleitet wird.

11. Verfahren nach einem der Ansprüche 1 bis 10, **dadurch gekennzeichnet, dass** die Rektifikationsvorrichtungen (R1) und (R2) unabhängig voneinander Packungskolonnen sind.

12. Verfahren nach einem der Ansprüche 1 bis 11, **dadurch gekennzeichnet, dass** Rektifikationsvorrichtung (R2) keinen Verdampfer hat.

13. Verfahren gemäß einem der Ansprüche 1 bis 12, **dadurch gekennzeichnet, dass** das im Roh- Adipodinitril (Roh-ADN) enthaltene ADN durch eine Umsetzung von Butadien mit Blausäure (HCN) hergestellt wird, wobei bei der ADN-Herstellung vorzugsweise in einem ersten Verfahrensschritt Butadien mit HCN in Gegenwart eines Nickel(0)-Phosphorligand-Komplexkatalysators (Ni-P-Kat) zu 3-Pentennitril (3-PN) umgesetzt wird, worauf in einem zweiten Verfahrensschritt 3-PN mit HCN in Gegenwart desselben Ni-P-Kat und einer Lewis-Säure zu ADN umgesetzt wird.

14. Verfahren gemäß einem der Ansprüche 1 bis 13, **dadurch gekennzeichnet, dass** das aufgereinigte ADN in Gegenwart eines Hydrierkatalysators, vorzugsweise in Gegenwart eines Raney-Nickel-Katalysators, zu Hexamethylendiamin (HMD) hydriert wird.

## Claims

1. A process for purifying adiponitrile (ADN) comprising the following steps a) to c):
a) introducing a stream (S1) comprising crude adiponitrile (crude ADN) into a rectification apparatus (R1),
b) separating off a gaseous stream (S2) via a first side draw of (R1), wherein the first side draw of (R1) is disposed below the introduction point for introducing stream (S1) into (R1) and wherein gaseous stream (S2) comprises ADN, and
c) introducing gaseous stream (S2) into a second rectification apparatus (R2), wherein an ADN stream (S7) depleted in high boilers (HB) is drawn off from (R2) as overhead product and a liquid stream (S6) enriched in high boilers (HB) is drawn off from (R2) as bottoms.

2. The process according to claim 1, further comprising at least one of steps d), e) or f):
d) separating off a stream (S3) via a second side draw of rectification apparatus (R1), wherein the second side draw of (R1) is disposed above the introduction point for introducing stream (S1) into (R1) and wherein stream (S3) comprises 1-amino-2-cyanocyclopentene (ACCP) and/or
e) separating off a stream (S5) as overhead product from rectification apparatus (R1), wherein stream (S5) comprises pentenenitriles (PNs), particularly 3-pentenenitrile (3-PN) and/or
f) separating off a stream (S4) from the bottom of rectification apparatus (R1), wherein stream (S4) comprises high boilers (HB).

3. The process according to either of claims 1 and 2, wherein the crude adiponitrile (crude ADN) in stream (S1) comprises adiponitrile (ADN), pentenenitriles (PNs), high boilers (HB), 1-amino-2-cyanocyclopentene (ACCP), 2-methylglutaronitrile (2-MGN) and 2-ethylsuccinonitrile (2-ESN).

4. The process according to any of claims 1 to 3, wherein
i) stream (S4) comprises high boilers and ADN and is liquid, and/or
ii) stream (S3) comprises ACCP, 2-MGN and 2-ESN and is liquid, and/or
iii) the gaseous stream (S2) comprises ADN, high boilers and no more than 500 ppm of PNs, ACCP, 2-MGN and/or 2-ESN, preference being given to a total amount of PNs, ACCP, 2-MGN and 2-ESN in (S2) of no more than 500 ppm, more preferably no more than 200 ppm, more particularly no more than 100 ppm.

5. The process according to any of claims 1 to 4, wherein a stream (S8) comprising crude ADN and high boilers (HB) is introduced into an evaporation apparatus (E3), wherein a stream (S1) depleted in non-volatile high boilers (HB) compared to stream (S8) is discharged from (E3) and passed into rectification apparatus (R1), and wherein a stream (S9) which comprises non-volatile high boilers and is depleted in ADN compared to stream (S8) is discharged from (E3).

6. The process according to claim 5, wherein stream (S9) is discharged from the bottom of evaporation apparatus (E3) and passed into an evaporation apparatus (E4), wherein a stream (S10) depleted in non-volatile high boilers compared to stream (S9) is discharged from (E4) and passed into rectification apparatus (R1) and/or returned to evaporation apparatus (E3), and wherein a stream (S11) depleted in ADN compared to stream (S9) is discharged from (E4).

7. The process according to any of claims 1 to 6, wherein some or all of stream (S4) is passed from rectification apparatus (R1) into evaporation apparatus (E3) and/or into evaporation apparatus (E4) .

8. The process according to any of claims 1 to 7, wherein gaseous stream (S2) is introduced into the bottom of (R2) and pure ADN is drawn off from (R2) as overhead product.

9. The process according to any of claims 1 to 8, wherein the ADN drawn off from (R2) as overhead product has a purity of at least 99.0%, preferably at least 99.5%, more preferably at least 99.8% and/or the total amount of PNs, ACCP, 2-MGN and 2-ESN in the ADN drawn off from (R2) as overhead product is no more than 500 ppm, more preferably no more than 200 ppm, more particularly no more than 100 ppm.

10. The process according to any of claims 1 to 9, wherein stream (S6) comprising high boilers (HB) and ADN is drawn off from the bottom of (R2) and some or all of said stream is returned to the bottom of rectification apparatus (R1).

11. The process according to any of claims 1 to 10, wherein rectification apparatuses (R1) and (R2) are each independently columns with structured packing.

12. The process according to any of claims 1 to 11, wherein rectification apparatus (R2) does not have an evaporator.

13. The process according to any of claims 1 to 12, wherein the ADN present in the crude adiponitrile (crude ADN) is produced by a reaction of butadiene with hydrocyanic acid (HCN), wherein the ADN production process preferably comprises a first step of reacting butadiene with HCN in the presence of a nickel(0) phosphorous ligand catalyst complex (NiP cat) to afford 3-pentenenitrile (3-PN) and a second step of subsequently reacting 3-PN with HCN in the presence of the same Ni-P cat and a Lewis acid to afford ADN.

14. The process according to any of claims 1 to 13, wherein the purified ADN is hydrogenated to afford hexamethylenediamine (HMD) in the presence of a hydrogenation catalyst, preferably in the presence of a Raney nickel catalyst.

## Revendications

1. Procédé pour la purification d'adipodinitrile (ADN), comprenant les étapes suivantes a) à c)
a) injection d'un flux (S1) contenant de l'adipodinitrile brut (ADN brut) dans un dispositif de rectification (R1),
b) séparation d'un flux gazeux (S2) via un premier soutirage latéral de (R1), le premier soutirage latéral de (R1) se trouvant sous le site d'injection du flux (S1) après (R1) et le flux gazeux (S2) contenant de l'ADN et
c) injection du flux gazeux (S2) dans un deuxième dispositif de rectification (R2), un flux d'ADN (S7) appauvri en fraction à point d'ébullition élevé (HS) étant soutiré de (R2) via la tête et un flux liquide (S6) enrichi en fraction à point d'ébullition élevé (HS) étant soutiré de (R2) via le fond.

2. Procédé selon la revendication 1, comprenant en outre au moins une des étapes d), e) ou f) :
d) séparation d'un flux (S3) via un deuxième soutirage latéral du dispositif de rectification (R1), le deuxième soutirage latéral de (R1) se trouvant au-dessus du site d'injection du flux (S1) après (R1) et le flux (S3) contenant du 1-amino-2-cyanocyclopentène (ACCP) et/ou
e) séparation d'un flux (S5) via la tête du dispositif de rectification (R1), le flux (S5) contenant des pentènenitriles (PNs), en particulier du 3-pentènenitrile (3-PN), et/ou
f) séparation d'un flux (S4) du fond du dispositif de rectification (R1), le flux (S4) contenant une fraction à point d'ébullition élevé (HS).

3. Procédé selon la revendication 1 ou 2, **caractérisé en ce que** l'adipodinitrile brut (ADN brut) dans le flux (S1) contient de l'adipodinitrile (ADN), des pentène-nitriles (PNs), une fraction à point d'ébullition élevé (HS), du 1-amino-2-cyanocyclopentène (ACCP), du 2-méthylglutaronitrile (2-MGN) et du 2-éthylsuccinonitrile (2-ESN).

4. Procédé selon l'une quelconque des revendications 1 à 3, **caractérisé en ce que**
i) le flux (S4) contient une fraction à point d'ébullition élevé et de l'ADN et est liquide et/ou
ii) le flux (S3) contient de l'ACCP, du 2-MGN et du 2-ESN et est liquide et/ou
iii) le flux gazeux (S2) contient de l'ADN, une fraction à point d'ébullition élevé et pas plus de 500 ppm de PNs, d'ACCP, de 2-MGN et/ou de 2-ESN, de préférence, la quantité totale de PNs, d'ACCP, de 2-MGN et de 2-ESN dans (S2) n'est pas supérieure à 500 ppm, plus préférablement pas supérieure à 200 ppm, en particulier pas supérieure à 100 ppm.

5. Procédé selon l'une quelconque des revendications 1 à 4, **caractérisé en ce qu'**un flux (S8), qui contient de l'ADN brut et une fraction à point d'ébullition élevé (HS), est introduit dans un dispositif d'évaporation (V3), un flux (S1), qui est appauvri en fraction à point d'ébullition élevé (HS) non volatile par rapport au flux (S8), étant évacué de (V3) et guidé dans le dispositif de rectification (R1) et un flux (S9), qui contient une fraction à point d'ébullition élevé non volatile et qui est appauvri en ADN par rapport au flux (S8), étant évacué de (V3).

6. Procédé selon la revendication 5, **caractérisé en ce que** le flux (S9) est évacué du fond du dispositif d'évaporation (V3) et guidé dans un dispositif d'évaporation (V4), un flux (S10), qui est appauvri en fraction à point d'ébullition élevé non volatile par rapport au flux (S9), étant évacué de (V4) et guidé dans le dispositif de rectification (R1) et/ou recyclé dans le dispositif d'évaporation (V3) et un flux (S11), qui est appauvri en ADN par rapport au flux (S9), étant évacué de (V4).

7. Procédé selon l'une quelconque des revendications 1 à 6, **caractérisé en ce que** le flux (S4) provenant du dispositif de rectification (R1) est guidé totalement ou partiellement dans le dispositif d'évaporation (V3) et/ou dans le dispositif d'évaporation (V4).

8. Procédé selon l'une quelconque des revendications 1 à 7, **caractérisé en ce que** le flux gazeux (S2) est injecté dans le fond de (R2) et de l'ADN pur est soutiré de la tête de (R2).

9. Procédé selon l'une quelconque des revendications 1 à 8, **caractérisé en ce que** la pureté de l'ADN soutiré de la tête de (R2) est d'au moins 99,0%, de préférence d'au moins 99,5%, de manière particulièrement préférée d'au moins 99,8% et/ou la quantité totale de PNs, d'ACCP, de 2-MGN et de 2-ESN dans l'ADN soutiré de la tête de (R2) n'est pas supérieure à 500 ppm, plus préférablement pas supérieure à 200 ppm, en particulier pas supérieure à 100 ppm.

10. Procédé selon l'une quelconque des revendications 1 à 9, **caractérisé en ce que** le flux (S6), qui contient une fraction à point d'ébullition élevé (HS) et de l'ADN, est soutiré du fond de (R2) et totalement ou partiellement recyclé dans le fond du dispositif de rectification (R1).

11. Procédé selon l'une quelconque des revendications 1 à 10, **caractérisé en ce que** les dispositifs de rectification (R1) et (R2) sont, indépendamment l'un de l'autre, des colonnes à garnissage.

12. Procédé selon l'une quelconque des revendications 1 à 11, **caractérisé en ce que** le dispositif de rectification (R2) ne présente pas d'évaporateur.

13. Procédé selon l'une quelconque des revendications 1 à 12, **caractérisé en ce que** l'ADN contenu dans l'adipodinitrile brut (ADN brut) est préparé par transformation de butadiène avec de l'acide cyanhydrique (HCN), où, lors de la préparation de l'ADN, on transforme de préférence dans une première étape de procédé du butadiène avec du HCN en présence d'un catalyseur complexe à base de nickel(0)-ligand phosphoré (Ni-P-Cat) en 3-pentène-nitrile (3-PN), suite à quoi, dans une deuxième étape de procédé, on transforme le 3-PN avec du HCN en présence du même Ni-P-Cat et un acide de Lewis en ADN.

14. Procédé selon l'une quelconque des revendications 1 à 13, **caractérisé en ce que** l'ADN purifié est hydrogéné en présence d'un catalyseur d'hydrogénation, de préférence en présence d'un catalyseur de type nickel de Raney, en hexaméthylènediamine (HMD).
